# EUROPEAN PATENT SPECIFICATION

(11) **EP 2 430 945 B1**
(45) Date of publication and mention of the grant of the patent: **11.06.2014**
(21) Application number: 11425205.9
(22) Date of filing: 29.07.2011
(51) Int. Cl.: A45D 34/04, A61N 1/30, A61N 1/32, A61N 1/04

(54) **Portable ion generator applicator device of cosmetic substannces to the eye area**
Tragbare Ionengenerator-Auftragungsvorrichtung für kosmetische Substanzen auf die Augenpartie
Dispositif portable générateur d'ions pour l'application de substances cosmétiques au niveau des yeux

(30) Priority: 17.09.2010 CN 201020533334 U
(43) Date of publication of application: 21.03.2012
(73) Proprietor: Laser.Com S.r.l., 35127 Padova (IT)
(72) Inventor: Chengxiu, Lu, 35127 Padova (IT); Casale, Antonio, 35013 Cittadella Padova (IT)
(74) Representative: Pulieri, Gianluca Antonio

(56) References cited:
- GB-A- 2 372 705
- JP-A- 8 266 328
- US-A1- 2005 191 252
- US-A1- 2010 191 171

## Description

The present invention relates to a portable ion generator device, for applying cosmetic substances, in particular for the eye area.

Applicator devices of cosmetic substances to the eye area exist in the prior art, in the form of pens or similar portable devices inside which skin treatment substances are inserted.

The known devices have several drawbacks.

For example, such devices are not particularly efficient and/or fast in permitting the application of the cosmetic substance and its absorption by the skin, in particular around the eye area.

In particular, no portable ion generator devices exist which permit an efficient and complete application of the cosmetic substances.

Moreover, once the cosmetic substances are finished the known devices are thrown away.

The problem which the present invention sets out to resolve is to make a device which overcomes the drawbacks mentioned in relation to the prior art.

Such drawbacks and limitations are resolved by a device according to claim 1.

The document US 2010/0191171 A1 discloses an applicator of the underlying context.

Other embodiments of the device according to the invention are described in the subsequent claims.

Further characteristics and advantages of the present invention will be more clearly comprehensible in the description below of a preferred and non-limiting embodiment, wherein:

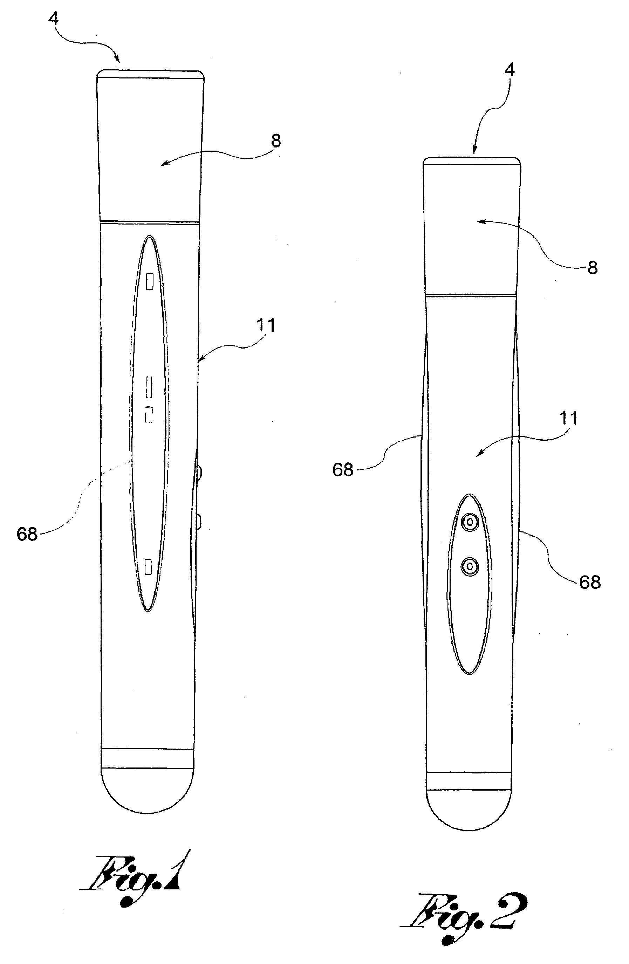

figures 1-2 show side views, from different angles, of a device according to one embodiment of the present invention;

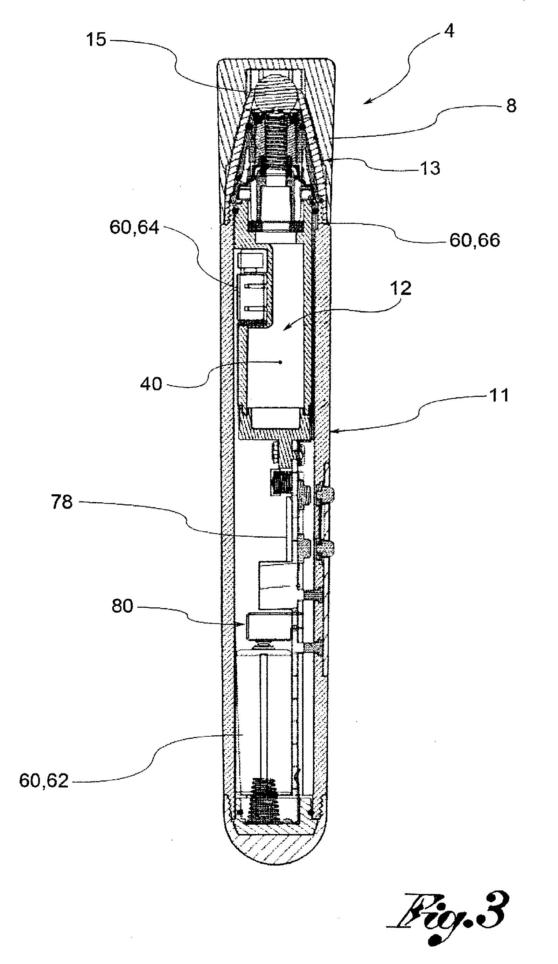

figure 3 shows a cross-section view of a device according to the present invention;

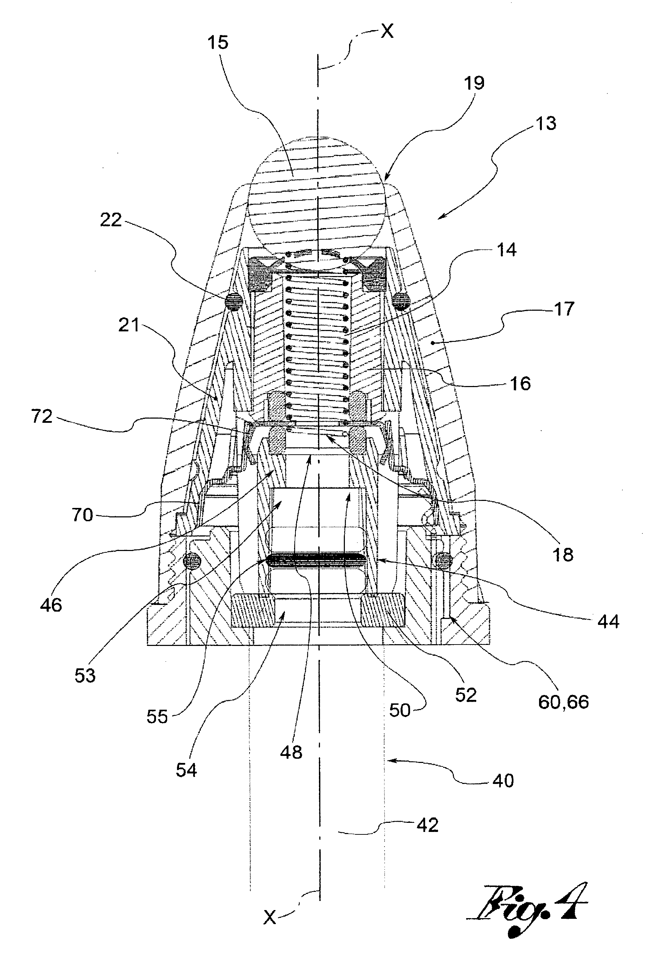

figure 4 shows a cross-section view of the tip of a device according to the present invention, in which the vial has been omitted;

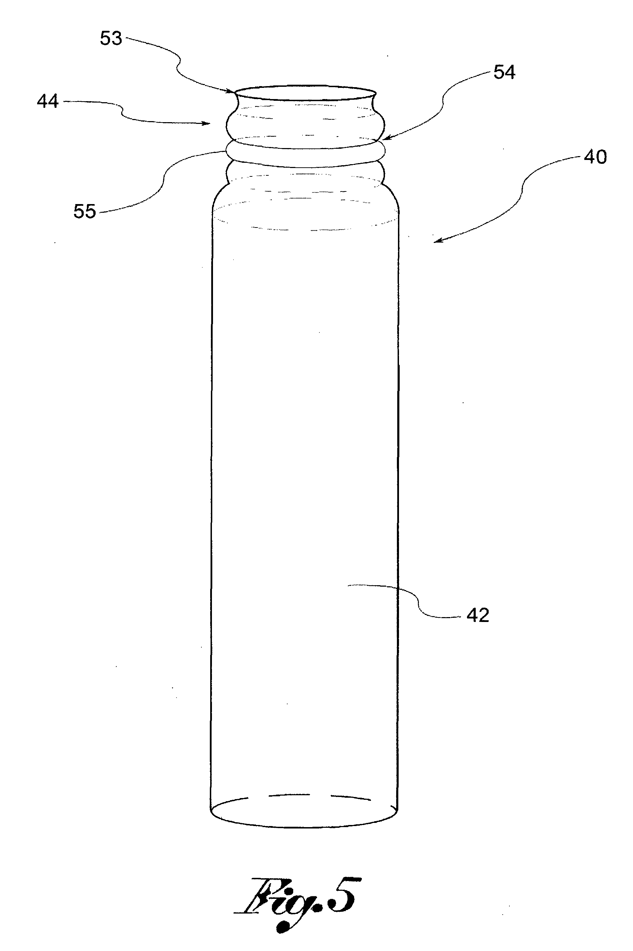

figure 5 shows a view of a vial, applicable to the device according to the invention.

With reference to the aforesaid drawings, reference numeral 4 globally denotes a portable ion generator applicator device of cosmetic substances, in particular for the eye area.

The use of the device 4 for the eye area is merely an example and should in no way be considered limiting of the possible uses of the same. In other words, the device 4 may be used for the application of the cosmetic substance to any part of the body.

The device 4 comprises a body 11, preferably of an elongated shape, along a main extension axis X―X, suitable to permit easy gripping of the device 4.

The body 11 defines a chamber 12, that is to say the space reserved for housing a vial 40 containing the cosmetic fluid or substance, as described further below.

In addition, a head 13 suitable to interface with the user is joined to the body 11. In particular, the head 13 comprises a roller 15, such as for example a sphere or cylinder, preferably in metal. Preferably, said roller 15 is covered by a removable cap 8 which protects the roller 15 and keeps the head 13 of the device 4 clean.

Said roller 15 is fitted onto a support ring 16 by the interposition of elastic means 14, such as a coil spring. Moreover, the roller 15 is blocked in position by a tapered portion 17 which the head 13 ends with. The tapered portion 17 has an aperture 19 having a smaller diameter than that of the roller 15 so as to prevent the roller 15 from coming off the head 13.

In particular, the support ring 16 is preferably made of metal and is coaxially positioned in the body 11 in relation to the main extension axis X-X.

The support ring 16 is hollow so as to define at least partially an outflow duct 18 of the cosmetic substances to be applied.

Preferably, the support ring 16 houses said elastic means 14 which support the roller 15 internally. Thanks to the elastic means 14 the roller has a stroke, parallel to the main extension axis X-X, so as to be able to dispense the fluid if subjected to compression between the skin of the user and the head 13. In other words in a rest condition the elastic means 14 push the rollex 15 so to come into contact with the aperture 19 of the tapered portion 17: thereby preventing exit of the liquid when the roller 15 is not pressed in contact with the skin.

However, if the roller 15 is as said pressed against the skin, then it moves backwards thanks to the compression of the elastic means 14 thereby permitting the dispensing of the substance thanks also to the rolling of said roller.

According to one embodiment, the tip 13 also comprises a support element 21, coaxial in relation to the support ring 16. Preferably, between said support element 21 and the tapered portion 17 at least one gasket 22, such as an O-ring 22, is positioned, to form a seal to the cosmetic substances.

As mentioned above, the device 4 comprises a vial 40 containing the cosmetic fluid or substances. The vial 40 comprises a tank 42 for the substance and an attachment portion 44 which permits the engagement or coupling of the same to the inside of the chamber 12.

Inside the chamber 12 there is a mouth portion 46, substantially cup-shaped, which defines a space suitable to receive said attachment portion 44 of the vial 40. The mouth portion 46 also comprises a hole 48 which is fluidically connected to the outflow duct 18. This way the substance coming out of the vial 40 passes through the hole 48 and the outflow duct 18 to reach as far as the roller 15.

The mouth portion 46 comprises a shoulder 50 so as to form a stop to the axial insertion of the attachment portion 44 of the vial 40.

A collar 52, suitable to form a shaped, preferably snap-coupling, is inserted inside the chamber, with a corresponding groove 54 made on the attachment portion 44 of the vial 40.

The axial length of the attachment portion of the vial 40 is such that when an axial extremity 53 of the attachment portion 44 abuts against the shoulder 50, the collar 52 engages in the groove 54. This way the insertion of the attachment portion 44 is strengthened and is more resistant to any detachments of the vial 40. To ensure the hermetic seal of the vial, at least one gasket 55 is inserted on the attachment portion 44, for example of the o-ring type: such gasket 55 ensures tightness between the attachment portion 44 and the inner lateral wall of the mouth portion 46.

The device comprises ion generator means 60 suitable to ionise the cosmetic substances to facilitate their cutaneous absorption. In particular, said ion generator means 60 comprise a battery 62 powering a first and second electric contact 64, 66.

The first electric contact 64 is, for example, connected to a first electric pole of the battery 62. Said first electric pole is in turn electrically connected to the user holding the device, for example by means of a metal plate or metal cheek 68 positioned outside the body 11 and connected to said first electric contact 64.

The second electric contact 66 is connected to a second electric pole of the battery 62.

The choice of the electric poles of the battery to connect to the respective electric contacts 64, 66 of the ion generator means 60 may be dual, depending on requirements and should not be considered reductive for the purposes of the sphere of protection of the present invention.

In other words it is possible to connect the first electric contact 64 to the negative electric pole of the battery 62 and the second electric contact 66 to the positive electric pole of the battery 62; alternatively the connections may be inverted so as to connect the first electric contact 64 to the positive electric pole of the battery 62 and the second electric contact 66 to the negative electric pole of the battery 62.

The second electric contact 66 has the function of ionising the cosmetic substance during its exit path from the vial 40.

In fact, the second electric contact 66 is electrically connected to conductor elements 70, 72, for example ribbon shaped, and positioned coaxially to the vial 40. Said conductor elements 70, 72 are also in electric contact with the support ring 16 which is in metal: the cosmetic substance is ionised in this before being applied to the skin.

Consequently, as described further below, the device applies the cosmetic substances by making use of electrophoresis and ionophoresis principles.

Obviously, inside the device there is an electric controller card 78 which controls and manages the device functions.

According to one possible embodiment, the device further comprises motor means 80 suitable to create a vibration to transmit to the device: in other words, the motor 80 activates the vibrating function of micro-massage. For example, said motor means comprise a rotating or translating mass so as to generate vibrations which are transmitted to the body of the device and thence to the tip. Such vibrations favour the application and absorption of the cosmetic substances: in addition such vibrations create an effect of slight anaesthesia on the part of the body to be treated, improving the comfort of the applicator device. This is an important aspect for application to particularly sensitive areas of skin, such as around the eyes.

The functioning of the device according to the present invention will now be described.

In particular, the substance is applied by placing and rotating the roller tip against the skin, so that by pressing the springs the liquid is released.

Contemporarily to the mechanical application of the substance from the roller tip, the absorption of the cosmetic substance is optimised by the application of electric fields created by the electric contacts connected to the battery.

In particular, the principle of electrophoresis is exploited which is an analytical method based on the movement of electrically charged particles immersed in a fluid caused by an electric field applied by means of a pair of electrodes to said fluid. The particles move towards the cathode if they have a positive charge and towards the anode if they have a negative charge.

Consequently, if the second electric contact 66 is connected to the positive pole of the battery 62 and the first electric contact 64 is connected to the negative pole of the battery 62, the particles of the cosmetic substances are charged positively and migrate towards the negative pole, that is towards the user who, by holding the device is negatively charged thanks to the dedicated plates 68 positioned outside the body of the device; in other words the particles of cosmetic substances, positively charged, migrate towards the user's skin which is negatively charged.

Vice versa, if the second electric contact 66 is connected to the negative pole of the battery 62 and the first electric contact 64 is connected to the positive pole of the battery 62, the particles of the cosmetic substances are charged negatively and migrate towards the positive pole, that is towards the user who, by holding the device is positively charged thanks to the dedicated plates 68 positioned outside the body of the device; in other words the particles of cosmetic substances, negatively charged, migrate towards the user's skin which is positively charged.

This way the cutaneous absorption of the substance is improved and facilitated.

Moreover, the principle of ionophoresis is also exploited, that is to say the introduction of a substance into the body through the epidermis (transcutaneous administration route), using a direct current (galvanic current): such direct current is in fact produced by the battery placed inside the device.

As may be appreciated from the description, the device according to the present invention makes it possible to overcome the drawbacks of the prior art presented.

The device emits positive or negative ions, in synergy with the formulation contained inside the vial to prevent and reduce wrinkles in the eye area: this way upon contact with the skin, the device permits a more complete absorption of the cosmetic substances.

The liquid formulation contained in the vial comes out through the roller, which can regulate the quantity of fluid to let out in proportion to the pressure with which it is pressed against the skin.

The device according to the present invention is extremely practical and easy to use and permits an improvement of the absorption of cosmetic substances in a practical manner, without the use of complex and expensive medical equipment.

The application of the substances using the principles of electrophoresis and ionophoresis occurs simply by holding the device and passing it directly over the area to be treated without the need for lengthy and expensive medical appointment.

Unlike the solutions of the prior art which provide for the use of disposable applicator devices, the present invention envisages use of a reusable portable device with disposable vial which is applied to the applicator tip each time. The insertion and coupling of the vial and the device tip is tight preventing the particularly annoying leakage of liquid given that the portable ion generator device is, as such, usually carried in a handbags, suitcases or the pocket of a garment.

As seen, the improvement of absorption is achieved by exploiting the principles of electrophoresis and ionophoresis.

The advantages of administering substances in this manner include among others the possibility of avoiding administration of the substances by a systemic route (oral, intramuscular, intra Venous).

Moreover, the substance can be applied directly to the part of the body affected by the pathology and only the active ingredient need be introduced, without carriers (excipients).

Lastly, thanks to the ionophoresis, the ions can bind to specific protoplasmatic proteins and hyperpolarise the nerve endings.

A person skilled in the art may make numerous modifications and variations to the applicator devices described above so as to satisfy contingent and specific requirements, all contained within the sphere of the invention as defined by the appended claims.

## Claims

1. Portable ion generator applicator device of cosmetic substances (4), in particular for the eye area, comprising
- a vial (40),
- a body (11) extending along a main extension axis (X-X), said body (11) defining a chamber (12) for housing said vial (40) containing the cosmetic fluid or substance to be applied, said vial (40) having a tank (42) for the substance and an attachment portion (44) which permits the engagement or coupling of the same to the inside of the chamber (12),
- a head (13) suitable to interface with the user by means of a roller (15) for the application of the cosmetic substance, the head being joined to the body (11),
- the head defining an outflow duct (18) which allows the transit of the cosmetic substance from the vial (40) to the roller (15),
- wherein the device comprises ion generator means (60) suitable to ionise the cosmetic substance to improve cutaneous absorption, said ion generator means (60) comprise a battery (62) which powers a first and second electric contact (64,66), wherein the first electric contact (64) is connected to a first electric pole of the battery (62) and is in turn electrically connected to the user holding the device, by means of a metal plate or metal cheek (68) positioned outside the body (11) and connected to said first electric contact (64) and wherein the second electric contact (66) is connected to a second electric pole of the battery (62) and has the function of ionising the cosmetic substance during its exit path from the vial (40), said second electric contact (66) being electrically connected to conductor elements (70,72) positioned coaxially to the outflow duct (18), so as to ionise the cosmetic substance before it is applied to the skin , said cosmetic substance being applied exploiting the principles of electrophoresis and ionophoresis,
**characterised in that**
inside the chamber (12) there is a mouth portion (46), which defines a space suitable to receive said attachment portion (44) of the vial (40), the mouth portion (46) also comprising a hole (48) which is fluidically connected to the outflow duct (18), the mouth portion (46) comprising a shoulder (50) so as to form a stop to the axial insertion of the attachment portion (44) of the vial (40),
wherein a collar (52), suitable to form a shaped, preferably snap-coupling, is inserted inside the chamber, with a corresponding groove (54) made on the attachment portion (44) of the vial (40),
wherein the axial length of the attachment portion (44) of the vial (40) is such that when an axial extremity of the attachment portion (44) abuts against the shoulder (50), the collar (52) engages in the groove (54), wherein at least one gasket (55) is inserted on the attachment portion (44), said gasket (55) ensuring tightness between the attachment portion (44) and the internal side wall of the mouth portion (46).

2. Device (4) according to claim 1, wherein said roller (15) is made of metallic material.

3. Device (4) according to claim 1 or 2, wherein the roller (15) is fitted onto a support ring (16) by the interposition of elastic means (14) and is blocked in position by a tapered portion (17) which the head (13) ends with, the tapered portion (17) having an aperture (19) having a smaller diameter than that of the roller (15) so as to prevent the roller (15) from coming off the head (13).

4. Device (4) according to claim 3, wherein the conductor elements (70, 72) are in electric contact with the support ring (16).

5. Device (4) according to claim 3 or 4, wherein the support ring (16) is made from metal and is coaxially positioned in the body (11) in relation to the main extension axis (X-X).

6. Device (4) according to any of the claims from 3 to 5, wherein the support ring (16) is hollow so as to define at least partially the outflow duct (18) of the cosmetic substance to be applied.

7. Device (4) according to any of the claims from 3 to 6, wherein the support ring (16) houses said elastic means (14) supporting the roller (15) internally.

8. Device (4) according to any of the previous claims, wherein the device further comprises motor means (80) suitable to create a vibration to transmit to the device to facilitate the application and absorption of the cosmetic substance.

9. Device (4) according to claim 8, wherein said motor means (80) comprise a rotating or translating mass so as to generate vibrations which are transmitted to the body of the device and thence to the tip.

## Patentansprüche

1. Tragbare Ionengenerator-Auftragsvorrichtung für kosmetische Substanzen (4), insbesondere für die Augenpartie, umfassend
- eine Phiole (40),
- einen Körper (11), der sich entlang einer Haupterstreckungsachse (X-X) erstreckt, wobei der Körper (11) eine Kammer (12) zum Aufnehmen der Phiole (40) bestimmt, die das kosmetische Fluid oder die aufzutragende Substanz enthält, wobei die Phiole (40) einen Behälter (42) für die Substanz und einen Befestigungsabschnitt (44) aufweist, der erlaubt, den selben mit dem Inneren der Kammer (12) in Eingriff zu bringen oder zu koppeln,
- einen Kopf (13), der geeignet ist, mit dem Benutzer durch eine Rolle (15) zum Auftragen der kosmetischen Substanz in Verbindung zu treten, wobei der Kopf mit dem Körper (11) verbunden ist,
- wobei der Kopf einen Auslasskanal (18) bestimmt, der den Durchgang der kosmetischen Substanz von der Phiole (40) zu der Rolle (15) erlaubt,
- wobei die Vorrichtung Ionengeneratormittel (60) umfasst, die geeignet sind, die kosmetische Substanz zu ionisieren, um die kutane Resorption zu verbessern, wobei die Ionengeneratormittel (60) eine Batterie (62) umfassen, die einen ersten und zweiten elektrischen Kontakt (64,66) antreibt, wobei der erste elektrische Kontakt (64) mit einem ersten elektrischen Pol der Batterie (62) verbunden ist und dieser wiederum elektrisch mit dem Benutzer, der die Vorrichtung hält, durch eine Metallplatte oder Metallwange (68) verbunden ist, die außerhalb des Körpers (11) positioniert und mit dem ersten elektrischen Kontakt (64) verbunden ist, und wobei der zweite elektrische Kontakt (66) mit einem zweiten elektrischen Pol der Batterie (62) verbunden ist und die Funktion aufweist, die kosmetische Substanz während ihres Austritts aus der Phiole (40) zu ionisieren, wobei der zweite elektrische Kontakt (66) mit Teilleitern (70,72) elektrisch verbunden ist, die koaxial zum Auslasskanal (18) positioniert sind, um so die kosmetische Substanz zu ionisieren, bevor sie auf die Haut aufgetragen wird, wobei die kosmetische Substanz unter Ausnutzung des Prinzips der Elektrophorese und Ionophorese aufgetragen wird,
**dadurch gekennzeichnet, dass**
innerhalb der Kammer (12) ein Mündungsabschnitt (46) ist, der einen Raum bestimmt, der geeignet ist, den Befestigungsabschnitt (44) der Phiole (40) aufzunehmen, wobei der Mündungsabschnitt (46) auch ein Loch (48) umfasst, das in Fluidverbindung mit dem Auslasskanal (18) steht, wobei der Mündungsabschnitt (46) eine Schulter (50) umfasst, um so einen Anschlag für die axiale Einführung des Befestigungsabschnitts (44) der Phiole (40) zu bilden,
wobei ein Bund (52), geeignet um eine geformte, vorzugsweise eine Schnappkupplung zu bilden, in die Kammer eingeführt ist, wobei eine entsprechende Nut (54) auf dem Befestigungsabschnitt (44) der Phiole (40) gefertigt ist,
wobei die axiale Länge des Befestigungsabschnitts (44) der Phiole (40) so ist, dass wenn ein axiales äußeres Ende des Befestigungsabschnitts (44) an der Schulter (50) anliegt, der Bund (52) in die Nut (54) eingreift,
wobei mindestens eine Dichtung (55) in den Befestigungsabschnitt (44) eingeführt ist, wobei die Dichtung (55) die Dichte zwischen dem Befestigungsabschnitt (44) und der inneren Seitenwand des Mündungsabschnitts (46) sicherstellt.

2. Vorrichtung (4) nach Anspruch 1, wobei die Rolle (15) aus metallischem Werkstoff besteht.

3. Vorrichtung (4) nach Anspruch 1 oder 2, wobei die Rolle (15) durch das Einfügen von elastischen Mitteln (14) auf einem Stützring (16) angebracht ist und durch einen sich verjüngenden Abschnitt (17), in dem der Kopf (13) endet, in der Position blockiert ist, wobei der sich verjüngende Abschnitt (17) eine Öffnung (19) aufweist, die einen kleineren Durchmesser als den der Rolle (15) aufweist, um so zu verhindern, dass sich die Rolle (15) vom Kopf (13) löst.

4. Vorrichtung (4) nach Anspruch 3, wobei sich die Teilleiter (70, 72) in elektrischem Kontakt mit dem Stützring (16) befinden.

5. Vorrichtung (4) nach Anspruch 3 oder 4, wobei der Stützring (16) aus Metall besteht und koaxial in Bezug auf die Haupterstreckungsachse (X-X) in dem Körper (11) positioniert ist.

6. Vorrichtung (4) nach einem der Ansprüche 3 bis 5, wobei der Stützring (16) hohl ist, um so mindestens teilweise den Auslasskanal (18) der aufzutragenden kosmetischen Substanz zu bestimmen.

7. Vorrichtung (4) nach einem der Ansprüche 3 bis 6, wobei der Stützring (16) die elastischen Mittel (14), die die Rolle (15) stützen, im Innern aufnimmt.

8. Vorrichtung (4) nach einem der vorhergehenden Ansprüche, wobei die Vorrichtung überdies Motormittel (80) umfasst, die geeignet sind, eine Vibration zu erzeugen, die an die Vorrichtung übertragen wird, um den Auftrag und die Resorption der kosmetischen Substanz zu erleichtern.

9. Vorrichtung (4) nach Anspruch 8, wobei die Motormittel (80) eine Schwungmasse oder translatorisch bewegte Masse umfassen, um so Vibrationen zu erzeugen, die auf den Körper der Vorrichtung und danach auf die Spitze übertragen werden.

## Revendications

1. Dispositif portable générateur d'ions pour l'application de substances cosmétiques (4), en particulier pour la zone des yeux, comprenant
- un flacon (40),
- un corps (11) s'étendant le long d'un axe d'extension principal (X-X), ledit corps (11) définissant une chambre (12) pour abriter ledit flacon (40) contenant le fluide ou la substance cosmétique à appliquer, ledit flacon (40) ayant un réservoir (42) pour la substance et une partie de fixation (44) qui permet la mise en prise ou le couplage de celui-ci avec l'intérieur de la chambre (12),
- une tête (13) adaptée pour faire l'interface avec l'utilisateur au moyen d'un rouleau (15) pour l'application de la substance cosmétique, la tête étant jointe au corps (11),
- la tête définissant un conduit d'écoulement de sortie (18) qui permet le transit de la substance cosmétique depuis le flacon (40) au rouleau (15),
dans lequel le dispositif comprend un système générateur d'ions (60) adapté pour ioniser la substance cosmétique afin d'améliorer l'absorption cutanée, ledit système générateur d'ions (60) comprend une batterie (62) qui alimente un premier et un second contact électrique (64, 66), dans lequel le premier contact électrique (64) est raccordé à un premier pôle électrique de la batterie (62) et est à son tour électriquement raccordé à l'utilisateur qui tient le dispositif, au moyen d'une plaque métallique ou d'un flasque métallique (68) positionné hors du corps (11) et raccordé audit premier contact électrique (64) et dans lequel le second contact électrique (66) est raccordé à un second pôle électrique de la batterie (62) et a pour fonction d'ioniser la substance cosmétique pendant son chemin de sortie depuis le flacon (40), ledit second contact électrique (66) étant électriquement raccordé aux éléments conducteurs (70, 72) positionnés de manière coaxiale par rapport au conduit d'écoulement de sortie (18) de façon à ioniser la substance cosmétique avant qu'elle ne soit appliquée à la peau, ladite substance cosmétique étant appliquée en exploitant les principes de l'électrophorèse et de l'ionophorèse,
**caractérisé en ce que**,
à l'intérieur de la chambre (12), il existe une portion d'embouchure (46), qui définit un espace adapté pour recevoir ladite partie de fixation (44) du flacon (40), la portion d'embouchure (46) comprenant également un trou (48) qui est raccordé de manière fluidique au conduit d'écoulement de sortie (18), la portion d'embouchure (46) comprenant un épaulement (50) de façon à former une butée à l'insertion axiale de la partie de fixation (44) du flacon (40),
dans lequel un collier (52) adapté pour former un accouplement façonné, de préférence un accouplement rapide, est inséré à l'intérieur de la chambre, avec une rainure correspondante (54) réalisée sur la partie de fixation (44) du flacon (40),
dans lequel la longueur axiale de la partie de fixation (44) du flacon (40) est telle que, lorsqu'une extrémité axiale de la partie de fixation (44) vient en butée contre l'épaulement (50), le collier (52) se met en prise dans la rainure (54),
dans lequel au moins un joint (55) est inséré sur la partie de fixation (44), ledit joint (55) assurant l'étanchéité entre la partie de fixation (44) et la paroi latérale interne de la portion d'embouchure (46).

2. Dispositif (4) selon la revendication 1, dans lequel ledit rouleau (15) est constitué de matériau métallique.

3. Dispositif (4) selon la revendication 1 ou 2, dans lequel le rouleau (15) est installé sur une bague de support (16) par l'interposition de systèmes élastiques (14) et est bloqué en position par une partie conique (17) avec laquelle la tête (13) se termine, la partie conique (17) ayant une ouverture (19) ayant un diamètre inférieur à celui du rouleau (15) de façon à éviter que le rouleau (15) ne sorte de la tête (13).

4. Dispositif (4) selon la revendication 3, dans lequel les éléments conducteurs (70, 72) sont en contact électrique avec la bague de support (16).

5. Dispositif (4) selon la revendication 3 ou 4, dans lequel la bague de support (16) est constituée de métal et est positionnée de manière coaxiale dans le corps (11) relativement à l'axe d'extension principal (X-X).

6. Dispositif (4) selon l'une quelconque des revendications 3 à 5, dans lequel la bague de support (16) est creuse de façon à définir au moins partiellement le conduit d'écoulement de sortie (18) de la substance cosmétique à appliquer.

7. Dispositif (4) selon l'une quelconque des revendications 3 à 6, dans lequel la bague de support (16) abrite ledit système élastique (14) en supportant le rouleau (15) de manière interne.

8. Dispositif (4) selon l'une quelconque des revendications précédentes, dans lequel le dispositif comprend en outre un moyen moteur (80) adapté pour créer une vibration à transmettre au dispositif afin de faciliter l'application et l'absorption de la substance cosmétique.

9. Dispositif (4) selon la revendication 8, dans lequel ledit moyen moteur (80) comprend une masse rotative ou en translation de façon à générer des vibrations qui sont transmises au corps du dispositif et ensuite à la pointe.
